# FASCICULE DE BREVET EUROPEEN

(11) **EP 2 523 595 B1**
(45) Date de publication et mention de la délivrance du brevet: **22.08.2018**
(21) Numéro de dépôt: 11700172.7
(22) Date de dépôt: 12.01.2011
(51) Int. Cl.: A61B 5/00, A61F 2/02, A61M 5/172, G06F 19/00

(54) **CAPTEUR POUR LA MESURE DE L'ACTIVITÉ DES ÎLOTS DE LANGERHANS, FABRICATION ET UTILISATION D'UN TEL CAPTEUR**
SENSOR ZUR MESSUNG DER AKTIVITÄT LANGERHANS-INSELN, VERFAHREN ZU DESSEN HERSTELLUNG UND VERWENDUNG
SENSOR FOR MEASURING THE ACTIVITY OF ISLETS OF LANGERHANS, METHOD FOR PRODUCING AND USE OF THE SAME

(30) Priorité: 13.01.2010 FR 1050202
(43) Date de publication de la demande: 21.11.2012
(73) Titulaire: Université de Bordeaux 1, 33400 Talence (FR); Centre National de la Recherche Scientifique (CNRS), 75016 Paris (FR); Université Bordeaux Segalen, 33000 Bordeaux (FR); Institut Polytechnique de Bordeaux, 33400 Talence (FR)
(72) Inventeur: LANG, Jochen, 33000 Bordeaux (FR); CATARGI, Bogdan, 33200 Bordeaux (FR); RENAUD, Sylvie, 33600 Pessac (FR); RAOUX, Matthieu, 33000 Bordeaux (FR); CHARPENTIER, Gilles, 33700 Merignac (FR); BORNAT, Yannick, 33400 Talence (FR)
(74) Mandataire: Regimbeau
(86) Numéro de dépôt international: PCT/EP2011/050359
(87) Numéro de publication internationale: WO 2011/086105

(56) Documents cités:
- DE-A1- 3 941 873
- DE-A1- 4 002 559
- US-A- 4 633 878
- US-A- 5 050 612
- US-A- 5 116 494
- US-A1- 2006 057 771
- US-B2- 6 605 039
- Bornat Y. et al.: "Detection of electrical activity of pancreatic beta-cells using micro-electrode arrays" In: "Fifth IEEE International Symposium on Electronic Design, Test and Applications", 13 janvier 2010 (2010-01-13), IEEE Computer Society, XP002586590, ISBN: 978-0-7695-3978-2 pages 233-236, le document en entier
- LEBRETON F. ET AL.: "Slow potentials encode intercellular coupling and insulin demand in pancreatic beta cells", DIABETOLOGIA, vol. 58, no. 6, 19 March 2015 (2015-03-19) , pages 1291-1299, XP035495456,

## Description

### Domaine

L'invention concerne le domaine des capteurs, notamment mais non limitativement pour une utilisation pour un traitement du diabète par distribution d'insuline.

L'invention concerne également le domaine des dispositifs pouvant être implantables dans le corps d'un patient, et comprenant un distributeur à insuline pour distribuer une quantité d'insuline.

L'invention concerne également un procédé pour fabriquer un tel capteur et un tel dispositif et une utilisation d'un tel capteur.

### État de la technique

Le diabète sucré est dû à un taux de glucose dans le sang (glycémie) trop élevé. Ceci traduit l'incapacité de l'organisme d'un patient à métaboliser le glucose.

Le métabolisme du glucose dépend de l'insuline, une hormone secrétée par des cellules β-pancréatiques des îlots de Langerhans dans le pancréas.

Chez certains patients atteints de diabète sucré, les cellules β-pancréatiques sont soit détruites soit insuffisantes, et il n'y a plus de sécrétion d'insuline ou de sécrétion en quantité adéquate (diabète dit « de type 1 »).

Dans le cadre du diabète dit « de type 1 », le traitement consiste généralement à injecter des doses d'insuline dans le corps d'un patient, parfois plusieurs fois par jour, et à surveiller le régime alimentaire du patient.

Un nombre limité de personnes atteintes d'un autre type de diabète sucré dit « de type 2 » (les cellules du patient dont le rôle est de capter et d'utiliser le glucose grâce à l'insuline deviennent insensibles à l'insuline) ont également recours à l'injection d'insuline.

En outre des injections d'insuline, il faut également surveiller plusieurs fois par jour le taux de glucose dans le sang du patient, afin de vérifier qu'il n'est pas en état d'hypoglycémie, néfaste pour la santé du patient.

D'ailleurs, les besoins en insuline dépendent de l'état et de l'activité physique et intellectuelle de la personne et sont régulés par des hormones.

Afin de connaître la glycémie, il est généralement procédé comme suit.

Un doigt du patient est piqué par une aiguille et est pressé pour faire perler une goutte de sang. Une languette reliée à un dispositif dédié pour la détermination de la glycémie est appliquée sur la goutte de sang. Le dispositif indique, en général sur un écran LCD, le taux de glucose dans le sang (glycémie) chez le patient. Il est décidé ensuite s'il est procédé à une injection d'insuline. Cette opération est donc lourde.

C'est pourquoi une solution a été recherchée pour permettre un contrôle de la glycémie moins contraignant et un rythme d'injection d'insuline plus adapté aux besoins du patient.

De récents progrès permettent désormais le suivi continu de la glycémie.

Une solution consiste à introduire dans le corps du patient des capteurs électrochimiques comprenant des électrodes reliées à une enzyme, la glucose oxydase. La glucose oxydase réagit, en présence d'oxygène, avec le glucose présent dans le sang pour produire de l'eau oxygénée et de la gluconolactone. L'eau oxygénée est ensuite oxydée et transformée en eau par les électrodes, qui produisent alors un courant électrique proportionnel à la concentration de glucose dans le sang.

L'inconvénient majeur de cette solution est qu'elle ne permet pas de collecter des informations sur les besoins en insuline, à cause de la technologie utilisée qui détecte seulement la glycémie mais pas les autres régulateurs hormonaux du métabolisme du glucose et ne prend pas en compte l'homéostasie glucidique, c'est-à-dire la capacité de l'organisme à maintenir un équilibre glycémique permettant son fonctionnement en dépit de contraintes extérieures tendant à écarter l'organisme de cet équilibre. De plus, la collecte des informations ne peut se faire en temps réel.

Des solutions non invasives existent, telles que :
- le dosage par spectroscopie de Raman, amplifiée par la surface (« surface-enhanced Raman spectroscopy » en anglais) ;
- la spectroscopie par fluorescence ;
- l'iontophorèse inversée ;
- la spectroscopie photo-acoustique ;
- la spectroscopie thermale ou par impédance ; ou
- la mesure du champ électromagnétique.

Mais ces solutions non invasives ne sont pas adaptées à un usage en dehors d'une surveillance médicale constante, par exemple en milieu hospitalier.

Toutes les solutions conventionnelles précitées utilisent un seul paramètre, à savoir le taux de glucose dans le sang, pour déterminer le besoin en insuline et/ou en glucose, alors que d'autres composés tels que d'autres hormones, les lipides et certains acides aminés modulent le besoin en insuline et/ou en glucose de manière physiologique. Ainsi le taux de glucose dans le sang ne reflète pas toujours à lui seul le réel besoin en insuline et/ou en glucose. C'est pourquoi des algorithmes complexes tenant compte de la situation physiologique sont nécessaires pour pallier l'utilisation d'un seul paramètre.

De plus, de telles solutions donnent des résultats moins fiables lors de la détection des états d'hypoglycémie dangereux pour la santé du patient.

Enfin, elles ne permettent pas la distribution d'insuline de manière oscillatoire. Or la distribution d'insuline de manière oscillatoire permettrait d'éviter l'instauration d'une résistance à l'insuline, c'est-à-dire les cas dans lesquels, malgré la présence d'insuline, l'organisme du patient n'arrive pas à métaboliser le glucose.

Egalement, dans le domaine de la recherche sur les cellules β-pancréatiques, les études sur de nouveaux principes thérapeutiques ou d'agents toxiques sont relativement complexes et difficiles pour des approches par criblage. Ces études peuvent en outre nécessiter le recours au génie génétique, ce qui altère ainsi l'état natif des cellules β-pancréatiques.

### Présentation

L'objet de l'invention est donc de pallier au moins un des inconvénients ci-dessus.

L'objet de l'invention est également l'observation à long terme de l'état des îlots de Langerhans afin d'élucider l'effet de substances sur leur activité électrique.

On propose selon l'invention un capteur selon la revendication 1.

Un avantage d'utiliser des îlots de Langerhans est que le capteur peut donner une information plus proche de l'état physiologique du patient que les capteurs de glucose décrits ci-dessus.

Un autre avantage est qu'un tel capteur peut également servir à étudier des îlots *ex-vivo*, notamment pour l'étude des îlots en vue d'un criblage de molécules toxiques, de molécules à visée thérapeutique.

Un tel capteur permet également le suivi de cellules lors de leur différentiation à partir de cellules souches en cellules β-pancréatiques et leur structuration en îlots de Langerhans.

D'autres caractéristiques de ce capteur optionnelles et non limitatives sont reprises dans les revendications 2 à 10.

L'invention concerne un dispositif selon les revendications 11, 12 ou 13. L'invention concerne également un procédé pour fabriquer un tel capteur et un tel dispositif, selon la revendication 14. L'invention concerne aussi une utilisation d'un tel capteur, selon la revendication 15. Un avantage d'utiliser des îlots de Langerhans est que le capteur peut donner une information plus proche de l'état physiologique du patient que les capteurs de glucose décrits ci-dessus.

### Présentation des figures

D'autres caractéristiques, buts et avantages de l'invention apparaîtront à la lecture de la description détaillée qui suit, en référence aux dessins donnés à titre illustratif et non limitatif, parmi lesquels :
- la figure 1 est une illustration partielle du capteur, en vue de dessus, au niveau des électrodes et des cellules β-pancréatiques ou des îlots de Langerhans ;
- la figure 2 est un graphe montrant la réaction des cellules β-pancréatiques en présence de glucose, déclencheur de signaux électriques, et de diazoxide, agent pharmacologique connu pour son inhibition de la réponse au glucose au niveau des cellules β-pancréatiques ou des îlots de Langerhans ;
- la figure 3 est un graphe illustrant la variation de la réaction des cellules β-pancréatiques en présence de glucose à deux concentrations différentes ;
- la figure 4 est un graphe illustrant la réaction des cellules β-pancréatiques en présence de l'hormone incrétine GLP-1, amplificateur physiologique de la réponse au glucose;
- la figure 5 est un schéma représentant le dispositif de distribution d'insuline selon un mode de réalisation de l'invention ;
- la figure 6 est un schéma représentant le dispositif de distribution d'insuline selon un autre mode de réalisation de l'invention ;
- la figure 7 est un schéma représentant le dispositif de distribution d'insuline selon un mode de réalisation de l'invention ;
- la figure 8 est un schéma représentant le dispositif de distribution d'insuline selon un mode de réalisation de l'invention ;
- la figure 9 est un organigramme représentant un exemple de mise en oeuvre du procédé de fabrication d'un capteur.

Sur l'ensemble des figures, les éléments similaires portent des références numériques identiques.

### Description détaillée

### Capteur

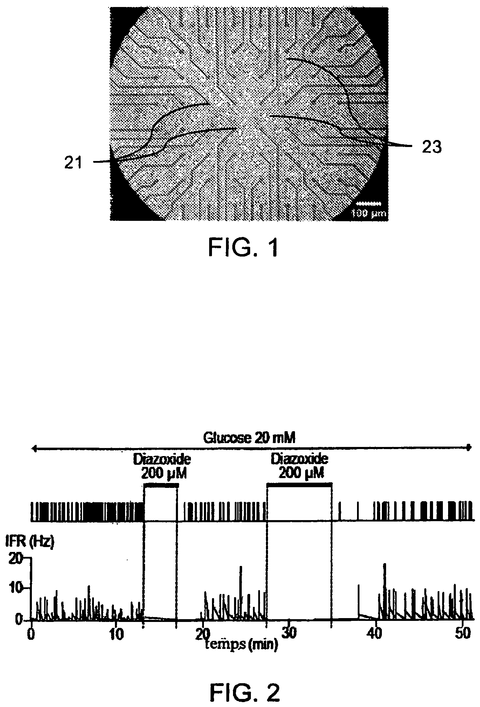

Un capteur **2** selon l'invention est décrit ci-après en référence aux figures 1, et 5 à 7.

Le capteur **2** peut être utilisé dans des dispositifs adaptés au traitement du diabète de type 1 ou 2, par distribution d'insuline.

Le capteur **2** comprend principalement un ensemble de microélectrodes **21** ; et des îlots **230** de Langerhans, contenant des cellules β-pancréatiques **23**, en culture sur l'ensemble de microélectrodes **21.**

L'ensemble de microélectrodes **21** est adapté pour mesurer en temps réel et en continu des signaux électriques **V** produits par les îlots **230** de Langerhans lors de leur activation physiologique.

Comme le montre la figure 1, la répartition des microélectrodes **21** sur le capteur **2** est telle que les microélectrodes **21** couvrent de façon homogène le capteur **2**, selon une géométrie régulière et conventionnelle. Les microélectrodes **21** sont espacées d'une distance comprise entre 50 et 200 µm. L'ensemble de microélectrodes **21** présente une densité de microélectrodes comprise entre 20 et 200 microélectrodes par millimètre carré.

Dans l'exemple de réalisation illustré sur la figure 1, le capteur **2** comprend un ensemble de 60 microélectrodes répartis sur 1 mm². Les microélectrodes **21** ont une forme de disque de 10 µm de diamètre.

Le capteur **2** comprend également des cellules β-pancréatiques **23** en culture sur l'ensemble de 60 microélectrodes **21.**

Le capteur **2** se distingue des capteurs utilisés dans des dispositifs conventionnels de traitement de diabète de type 1 ou 2 en ce qu'il ne mesure pas uniquement la glycémie. En effet, comme déjà précisé ci-dessus, les capteurs conventionnels utilisent la gluconolactone pour mesurer indirectement le glucose, dont le taux dans le sang est supposé refléter le besoin en insuline du patient, ce qui n'est pas forcément juste comme on l'a déjà vu.

L'utilisation de cellules β-pancréatiques permet de s'approcher du fonctionnement biologique naturel du pancréas d'un individu sain, que l'on cherche à reproduire chez un individu atteint d'un diabète de type 1 ou 2 (patient). En effet, les cellules β-pancréatiques régulent la glycémie en sécrétant l'insuline. Leur activation physiologique produit des signaux électriques, par exemple des potentiels électriques. Ainsi, l'activation physiologique d'une cellule β-pancréatique se traduit par une variation dynamique du potentiel électrique de part et d'autre de sa membrane. Cette variation dynamique a un profil d'amplitude en fonction du temps que l'on appelle une forme d'onde et qui reflète le comportement physiologique des cellules β-pancréatiques et alors le besoin en insuline du patient. Par exemple, il existe des profils particuliers que l'on appelle potentiels d'action connus de l'homme du métier et qui correspondent à une variation rapide et amplifiée du potentiel électrique au-delà d'un seuil de potentiel. Ces potentiels d'action peuvent être identifiés.

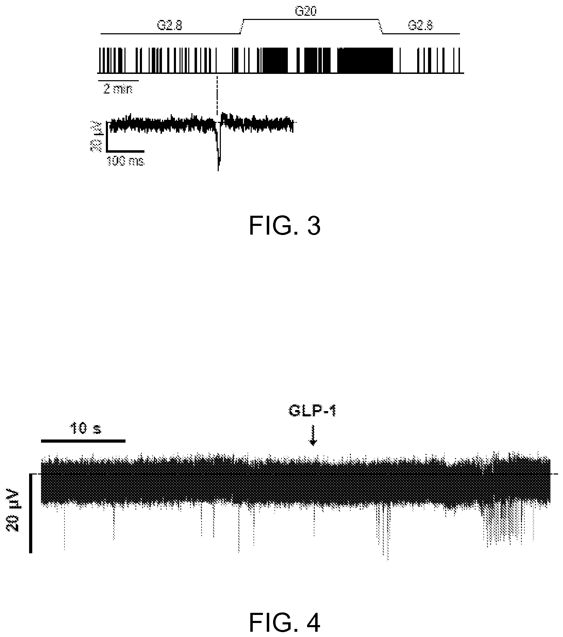

Dans le cas des cellules β-pancréatiques, la fréquence des potentiels d'action augmente avec la sécrétion d'insuline. Comme illustré par la figure 3, les potentiels électriques sont mesurés, en temps réel et en continue, i.e. dynamiquement, à l'échelle des millisecondes.

L'utilisation d'îlots de Langerhans permet de s'approcher encore plus du fonctionnement biologique naturel, puisque les cellules β-pancréatiques, représentant deux tiers des cellules des îlots de Langerhans, sont dans leur environnement naturel.

L'utilisation d'îlots de Langerhans conduit à l'obtention d'une information sur le besoin en insuline du patient, permettant une régulation de la glycémie chez celui-ci de manière proche d'une régulation de la glycémie chez un individu sain.

Le capteur **2** comprend, en outre, au moins un ensemble d'unités **25** de traitement. Chaque microélectrode **21** est alors couplée à une seule unité **25** de traitement pour, d'une part, la mise en forme des signaux électriques **V** mesurés, et d'autre part, l'extraction d'au moins un paramètre **P** traduisant un besoin en insuline.

Le paramètre **P** peut également traduire l'état d'activité électrique des îlots **230** de Langerhans pour une étude physiologique. Cette étude physiologique peut être effectuée dans le cadre d'un criblage de molécules toxiques ou d'un suivi de cellules pour étudier leur différenciation à partir de cellules souches en cellules β-pancréatiques et leur structuration en îlots de Langerhans. Cette étude physiologique peut encore être effectuée dans un but thérapeutique.

L'avantage de fournir une unité **25** de traitement par microélectrode **21** est de permettre le traitement en continu et en temps réel des signaux électriques **V** mesurés.

De préférence, les unités **25** de traitement sont des circuits conçus sur mesure en technologie microélectronique analogique, par exemple réalisés en technologie CMOS submicronique. Cette solution d'intégration microélectronique est optimale pour obtenir une forte densité d'intégration des circuits (quelques mm²) et une faible consommation d'énergie, permettant une plus longue durée d'utilisation du capteur **2** et limitant l'élévation de la température autour du capteur **2.** Le traitement analogique des signaux permet en effet de s'affranchir de la présence de calcul numérique (sur processeur standard ou dédié) et minimise donc les connexions internes au circuit en codant chaque signal sur un seul fil. De plus la réalisation des unités **25** de traitement en technologie CMOS standard est peu onéreuse.

L'unité **25** de traitement comprend une sous-unité **251** et une sous-unité **253.**

La mise en forme des signaux est réalisée par la sous-unité **251**, qui reçoit les signaux électriques **V** des microélectrodes **21** et les traite selon des fonctions mathématiques de type amplification et/ou filtrage. Dans le cas de la mesure de potentiels électriques, la sous-unité **251** peut détecter notamment les potentiels d'action.

L'extraction du paramètre **P** est réalisée par la sous-unité **253** d'extraction ; la valeur du paramètre **P** est calculée à partir de signaux d'entrée de la sous-unité **253** comportant les signaux électriques **V** mis en forme et une valeur **Vc** de consigne. La valeur **Vc** de consigne traduit une activation normale des cellules β-pancréatiques **23** ou des îlots **230** de Langerhans.

Le capteur **2** peut comprendre en outre un régulateur **26** pour transformer le paramètre **P** traduisant le besoin en insuline en un signal **C** de commande, pour commander un distributeur **3** d'insuline. Il y a un régulateur **26** pour l'ensemble des unités **25** de traitement. Le signal **C** de commande obtenu dépend de l'écart entre le paramètre **P** extrait par l'unité **25** de traitement et la valeur **Vc** de consigne, ainsi que de la dérivée de cet écart. Notamment, le signal **C** de commande dépend de la forme d'onde des signaux électriques **V** mis en forme - due au traitement temporel et statistique des signaux électriques **V** mesurées - et/ou des caractéristiques fréquentielles des signaux électriques **V** mis en forme. Le régulateur **26** tient compte également de consignes de sécurité telles que des limites relatives et absolues de la quantité d'insuline à distribuer et/ou une limitation des variations de la quantité d'insuline à distribuer.

Par exemple, le régulateur **26** peut mettre en oeuvre une détection à seuil variable en fonction de l'écart-type du signal électrique **V** mis en forme ou de la fréquence des oscillations ; un filtrage par ondelette ; etc.

L'ensemble de microélectrodes **21** et/ou l'ensemble d'unités **25** de traitement et/ou le régulateur **26** peuvent être réalisés ou reportés sur un matériau semi-conducteur, par exemple du silicium.

L'ensemble de microélectrodes **21** et/ou l'ensemble d'unités **25** de traitement et/ou le régulateur **26** peuvent être réalisés ou reportés sur le même support semi-conducteur ou sur des supports séparés, puis interconnectés électriquement en conséquence (grâce à des pistes métallisées **M** ou à des connections souples **F**).

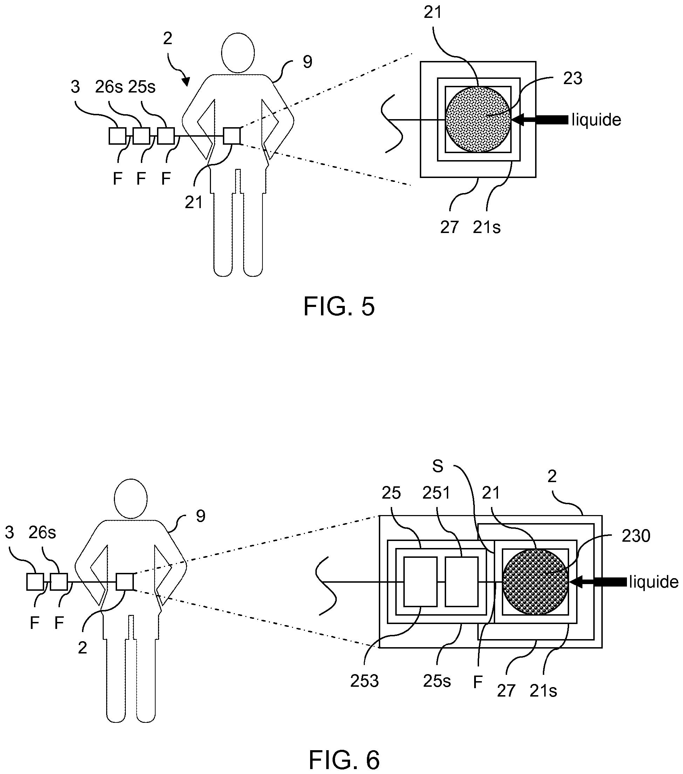

Par exemple, comme l'illustrent les figures 5 à 7, l'ensemble de microélectrodes **21**, l'ensemble d'unités **25** de traitement et le régulateur **26** sont réalisés sur des supports de silicium séparés **21s**, **25s**, **26s** et reliés par des connexions souples **F** (voir figures 5 à 7). Éventuellement les supports de silicium séparés **21s, 25s, 26s** peuvent être reportés sur un même support **28** puis connectés électriquement entre eux de manière connue de l'homme du métier (voir figure 6).

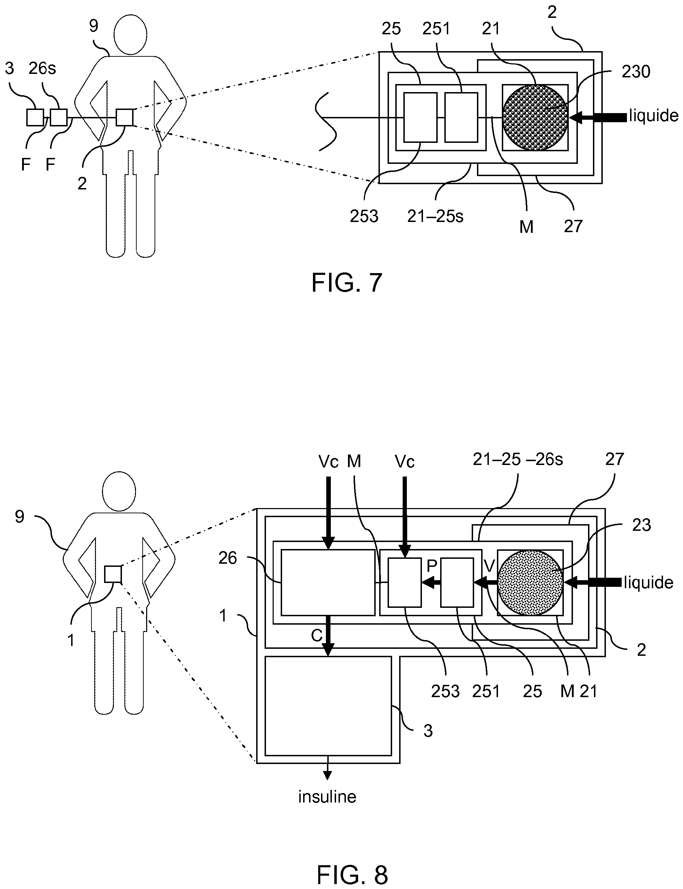

Dans l'exemple de la figure 7, l'ensemble de microélectrodes **21** et l'ensemble d'unités **25** de traitement sont réalisés directement sur le même support de silicium **21-25s** et sont reliés électriquement par des pistes métallisées **M.**

Dans l'exemple de la figure 8, l'ensemble de microélectrodes **21,** l'ensemble d'unités **25** de traitement et le régulateur **26** sont réalisés directement sur un même support de silicium **21-25-26s** et reliés électriquement par des pistes métallisées **M.**

L'avantage de réaliser l'ensemble d'unités **25** de traitement directement sur le support semi-conducteur est d'éviter la dégradation des signaux électriques **V** produits par les cellules β-pancréatiques **23** ou les îlots **230** de Langerhans et mesurés par l'ensemble de microélectrodes **21,** et la perte d'information liée à un traitement extracorporel des signaux électriques **V.**

La réalisation de l'ensemble de microélectrodes **21,** de l'ensemble d'unités **25** de traitement et du régulateur **26** sur des supports **21s, 23s, 25s, 21-25s, 21-25-26s** semi-conducteurs est déjà connue de l'homme du métier et ne sera pas décrite plus en détail par la suite. On citera un exemple de technologie : les réseaux de microélectrodes (ou MEA pour *microelectrode arrays* en anglais).

Le capteur **2** peut être adapté pour être implanté au moins partiellement dans le corps d'un patient **9** comme le montrent les figures 5 à 8. Dans le cas où le capteur **2** est partiellement implantable dans le corps du patient **9,** au moins l'ensemble de microélectrodes **21** et les cellules β-pancréatiques **23** ou îlots **230** de Langerhans sont à l'intérieur du corps du patient **9.** De préférence, l'ensemble d'unités **25** de traitement est aussi à l'intérieur du corps du patient **9** (figure 7). Il est possible également de réaliser un capteur **2** comprenant un ensemble de microélectrodes **21,** des cellules β-pancréatiques **23** ou îlots **230** de Langerhans, l'ensemble d'unité **25** de traitement et le régulateur **26,** et étant entièrement implantable dans le corps du patient **9** (figure 8).

L'implantation du capteur **2** dans le corps du patient **9** se fait de préférence dans le tissu interstitiel, près d'une artère ou du péritoine.

Les anticorps d'un organisme s'attaquent à toutes molécules reconnues comme étrangères ou non immunitairement compatibles avec l'organisme du patient **9,** comme c'est le cas pour les greffes.

Pour éviter ce rejet, le capteur **2** peut comprendre en outre une membrane **27** semi-perméable adaptée pour filtrer un liquide, par exemple le sang, devant accéder aux cellules β-pancréatiques **23** et bloquant toute molécule contenue dans le liquide dont le poids est supérieur à 65 kDa (kilodalton ; 1Da = 1,67·10⁻²⁴ g). Ainsi, il n'est pas besoin de choisir des cellules β-pancréatiques ou des îlots de Langerhans immunitairement compatibles avec le patient **9** : en présence de la membrane **27**, le risque de rejet est diminué et il n'est pas besoin de prescrire des immunodépresseurs.

L'alimentation en énergie électrique du capteur **2** peut être interne ou externe. Dans ce dernier cas, l'alimentation doit être mobile et pouvoir accompagner le patient **9** dans ses déplacements. L'alimentation externe peut être réalisée par induction extérieure. L'alimentation interne peut être réalisée par batterie.

Les cellules β-pancréatiques **23** ou les îlots **230** de Langerhans peuvent être d'origine porcine, murine (souris ou rats par exemple) ou humaine. Les cellules β-pancréatiques peuvent également être d'origine clonale (i.e. qu'elles ont été clonées à partir d'une ou plusieurs cellules β-pancréatiques) ou obtenues à partir de cellules souches (c'est-à-dire à partir de cellules non encore différenciées et pouvant se transformer en tout type de cellules). Les cellules β-pancréatiques humaines peuvent aussi être cadavériques, c'est-à-dire provenir de donneurs avant que leurs fonctions physiologiques ne soient affectées *post-mortem.*

La quantité de cellules β-pancréatiques **23** sur les microélectrodes **21** est comprise entre 0,01 et 1 % de la quantité de cellules β-pancréatiques dans le pancréas d'un individu sain. De préférence, la quantité de cellules β-pancréatique **23** est de 0,1 % de la quantité de cellules β-pancréatiques dans le pancréas d'un individu sain.

Le nombre d'îlots 230 de Langerhans est déterminé pour que la quantité de cellules β-pancréatiques contenues dans ces îlots 230 de Langerhans soit comprise entre 0,01 et 1 % de la quantité de cellules β-pancréatiques dans le pancréas d'un individu sain. De préférence, le nombre d'îlots 230 de Langerhans est déterminé pour que la quantité de cellules β-pancréatiques soit de 0,1 % de la quantité de cellules β-pancréatiques dans le pancréas d'un individu sain.

Le capteur **2** peut être utilisé à d'autres fins que l'insulinothérapie. En effet, le capteur **2** peut être utilisé pour divers tests tels que du criblage (test dans lequel on dépose plusieurs centaines voire milliers de composés sur au moins autant de capteurs) ou de l'analyse en laboratoire (mesure des besoins en glucose de manière extracorporelle par exemple).

### Résultats obtenus

Des tests ont été réalisés au laboratoire sur le capteur **2** de la figure 1.

La figure 2 illustre l'activation des cellules β-pancréatiques **23** du capteur **2** en présence de glucose et de diazoxide.

Il est connu que, *in vivo*, la présence de glucose entraîne une production de signaux électriques, par exemple sous forme de potentiels électriques dont le profil peut avoir la forme de potentiels d'action chez des cellules β-pancréatiques d'un individu sain. Les cellules β-pancréatiques libèrent alors de l'insuline. Il est également connu que, *in vivo*, la diazoxide inhibe l'effet du glucose sur les cellules β-pancréatiques d'un individu sain, c'est-à-dire qu'en présence conjointe du glucose et de la diazoxide, les cellules β-pancréatiques ne produisent pas de potentiel d'action et donc ne secrètent pas d'insuline.

Sur la première ligne de la figure 2, sont précisés les moments de présence de glucose à 20 mM, c'est-à-dire de 0 à 50 min. Sur la deuxième ligne, sont précisés les moments de présence de diazoxide à 200 µM, c'est-à-dire de 13 à 16 min environ et de 27 à 35 min environ. Sur la troisième ligne, chaque bâton représente l'occurrence d'un potentiel d'action identifié dans le signal électrique **V.** Sur la quatrième ligne, on a représenté la fréquence instantanée de ces potentiels d'action (IFR en hertz) en fonction du temps (en minute).

On peut voir que les cellules β-pancréatiques **23** du capteur **2** ont une activité physiologique soutenue en présence de glucose sans diazoxide, alors qu'en présence de diazoxide, les cellules β-pancréatiques **23** du capteur **2** ont une activité physiologique basale (activation des cellules β-pancréatiques **23** nulle).

On peut également voir que l'effet du diazoxide n'est pas définitif. En effet, dès qu'il n'y a plus de diazoxide, l'activation électrique des cellules β-pancréatiques **23** du capteur **2** redevient soutenue en présence de glucose seul.

Les cellules β-pancréatiques **23** du capteur **2** ont donc un comportement physiologique vis-à-vis du diazoxide.

La figure 3 montre la variation de l'occurrence de potentiels d'action identifiés dans le signal électrique **V** mesuré sur les cellules β-pancréatiques **23**, en fonction de la concentration de glucose. Sur la première ligne, on a représenté la concentration de glucose. Sur la deuxième ligne, chaque bâton représente l'occurrence d'un potentiel d'action. Sur la quatrième ligne est représenté le signal électrique **V** tel que mesuré dynamiquement en analogique et en continu. Une forme d'onde de potentiel d'action est visible à l'endroit du trait vertical en pointillé.

Il est visible sur la figure 3 que l'occurrence dans le signal électrique **V** de potentiels d'action est plus élevée lorsque la concentration de glucose est de 20 mM que lorsque la concentration de glucose est de 2,8 mM.

L'activité électrique des cellules β-pancréatiques **23** varie donc avec la concentration de glucose et l'occurrence des potentiels d'action augmente avec celle-ci, ce qui correspond à ce qui se passe pour des cellules β-pancréatiques du pancréas d'un individu sain.

La figure 4 illustre l'influence d'une hormone incrétine, la GLP-1, sur la production de potentiels d'action chez les cellules β-pancréatiques **23** du capteur **2.** Il est connu que pour un individu sain, l'hormone incrétine GLP-1 augmente la production de potentiels d'action chez les cellules β-pancréatiques et donc la sécrétion d'insuline.

Sur première ligne de la figure 4, est précisé l'instant où les cellules β-pancréatiques **23** du capteur **2** sont mises au contact de l'hormone incrétine GLP-1, représenté par une flèche verticale. Sur la deuxième ligne est représenté le signal électrique **V** mesuré dynamiquement en temps réel et en continu, avec les occurrences de potentiels d'action visibles sous forme de pics vers le bas.

On peut voir que l'activation électrique des cellules β-pancréatiques **23** du capteur **2** augmente fortement une vingtaine de secondes après l'injection de l'hormone incrétine GLP-1. Ceci reproduit bien ce qui est observé dans le milieu naturel des cellules β-pancréatiques du pancréas d'un individu sain.

### Procédé de fabrication d'un capteur

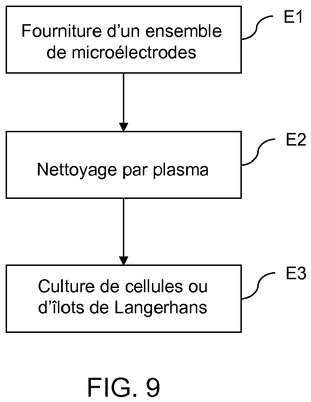

En référence à la figure 9, un procédé de fabrication du capteur **2** décrit ci-dessus est décrit ci-après.

Ce procédé comprend les étapes suivantes :
- fourniture **E1** d'un support **21s, 21-25s, 21-25-26s** présentant un ensemble de microélectrodes **21,** éventuellement un ensemble d'unités **25** de traitement et/ou un régulateur **26** ;
- nettoyage **E2** du support **21s, 21-25s, 21-25-26s** par plasma ; et
- culture **E3** de cellules β-pancréatiques **23** ou îlots **230** de Langerhans sur l'ensemble de microélectrodes **21.**

Le nettoyage **E2** du support par plasma (ou « *plasma cleaner »* en anglais) permet de rendre la surface du support **21s, 21-25s, 21-25-26s** hydrophobe et la prépare pour l'adhésion des cellules β-pancréatiques **23** sur le support **21s, 21-25s, 21-25-26s.**

L'obtention des cellules β-pancréatiques **23** ou îlots **230** de Langerhans est effectuée de manière conventionnelle.

### Dispositif pour la distribution d'insuline

Un exemple d'un dispositif 1 possible pour la distribution d'insuline est décrit ci-après en référence aux figures 5 à 8.

Ce dispositif **1** comprend un distributeur d'insuline **3** pour distribuer une quantité d'insuline dans le corps d'un patient **9.**

Le dispositif **1** comprend en outre un capteur **2** tel que décrit ci-dessus.

Le distributeur d'insuline **3** peut être implantable dans le corps du patient **9.** Ceci n'est pas obligatoire, le distributeur d'insuline **3** peut tout aussi bien être à l'extérieur du corps du patient **9.**

Dans le cas où le distributeur d'insuline **3** et le capteur **2** sont entièrement implantables dans le corps du patient **9**, le patient **9** ne sera pas gêné par la présence d'objet extérieur relié à son corps. De plus, ceci est avantageux tant sur les plans esthétique que psychologique. L'alimentation du dispositif **1** peut être identique à celle du capteur **2.**

La régulation de la glycémie est réalisée grâce à ce dispositif **2** en boucle fermée. En effet, les cellules β-pancréatiques **23** produisent des signaux électriques **V** en fonction du besoin en insuline du patient **9**, l'ensemble de microélectrodes **21** mesure ces signaux électriques **V** et l'ensemble d'unités **25** de traitement les met en forme et en extrait le paramètre **P.** Ce paramètre **P** est reçu par le régulateur **26** qui à partir de ce paramètre **P** génère et envoie un signal **C** de commande vers le distributeur d'insuline **3.** Le distributeur d'insuline **3** distribue la quantité d'insuline correspondant au signal **C** de commande. L'insuline distribuée agit sur la glycémie et modifie alors le besoin en insuline ultérieur du patient **9** et la diminution de la glycémie entraîne une diminution de l'activation des cellules β-pancréatiques **23** avec une diminution de la fréquence des potentiels d'action, donc un retour vers la ligne de base et arrêt du signal **C** de commande jusqu'à une prochaine augmentation de la glycémie, par exemple lors de la prise d'un repas.

## Revendications

1. Capteur (2) comprenant :
- un ensemble de microélectrodes (21);
**caractérisé en ce qu'**il comprend également :
- des îlots (230) de Langerhans en culture sur l'ensemble de microélectrodes (21) ;
l'ensemble de microélectrodes (21) étant adapté pour mesurer dynamiquement, en temps réel et en continu, des signaux électriques (V) produits par les îlots (230) de Langerhans lors de leur activation physiologique,
et **en ce qu'**il comprend en outre au moins un ensemble d'unités (25) de traitement, chaque unité (25) de traitement étant couplée à une microélectrode (21) et adaptée pour
- la mise en forme des signaux électriques (V) mesurés ; et
- l'extraction d'au moins un paramètre (P) traduisant un besoin en insuline d'un patient (9), en temps réel et en continu.

2. Capteur (2) selon la revendication 1 dans lequel chaque unité (25) de traitement comprend une sous-unité (251) adaptée à réaliser la mise en forme des signaux électriques (V), la sous-unité 251 étant adaptée pour recevoir les signaux électriques (V) et les traiter selon des fonctions mathématiques de type amplification et/ou filtrage.

3. Capteur (2) selon l'une des revendications 1 à 2 dans lequel les unités (25) de traitement sont des circuits conçus sur mesure en technologie microélectronique analogique.

4. Capteur (2) selon l'une des revendications précédentes, dans lequel chaque unité (25) de traitement comprend une sous-unité (253) adaptée pour réaliser l'extraction du paramètre (P), la valeur du paramètre étant calculée à partir de signaux d'entrée de la sous-unité (253) comportant les signaux électriques (V) mis en forme et une valeur (Vc) de consigne.

5. Capteur (2) selon l'une des revendications 1 à 4, dans lequel l'ensemble de microélectrodes (21) est adapté pour mesurer des signaux électriques sous forme de potentiels électriques.

6. Capteur (2) selon l'une des revendications 1 à 5, dans lequel l'unité (25) de traitement est adaptée pour détecter un potentiel d'action.

7. Capteur (2) selon l'une des revendications 1 à 6, comprenant en outre un régulateur (26) pour transformer le paramètre (P) traduisant le besoin en insuline en un signal (C) de commande pour commander un distributeur (3) d'insuline.

8. Capteur (2) selon l'une des revendications 1 à 7, comprenant en outre un support (21s, 21-25s, 21-25-26s) semi-conducteur comportant au moins l'ensemble de microélectrodes (21).

9. Capteur (2) selon l'une des revendications 1 à 8, comprenant en outre une membrane (27) semi-perméable adaptée pour
- filtrer un liquide devant accéder aux îlots (230) de Langerhans, et
- bloquer des molécules dudit liquide dont le poids est supérieur à 65 kDa.

10. Capteur (2) selon l'une des revendications 1 à 9, dans lequel les îlots de Langerhans (230) sont d'origine porcine, murine, ou humaine.

11. Dispositif (1) comprenant un distributeur (3) d'insuline pour distribuer une quantité d'insuline dans le corps d'un patient (9),
le dispositif (1) étant **caractérisé en ce qu'**il comprend en outre un capteur (2) selon l'une des revendications 1 à 10.

12. Dispositif (1) selon la revendication 11 dans lequel le capteur (2) comprend un régulateur (26) pour transformer le paramètre (P) traduisant le besoin en insuline en un signal (C) de commande, pour commander le distributeur (3) d'insuline.

13. Dispositif (1) selon la revendication 12 dans lequel l'ensemble de microélectrodes (21), le régulateur (26) et l'ensemble d'unité (25) sont réalisés ou reportés sur un même support semi-conducteur.

14. Procédé de fabrication d'un capteur (2) selon l'une des revendications 1 à 10 **caractérisé en ce qu'**il comprend les étapes suivantes :
- fourniture (E1) d'un support (21s, 23-25s, 23-25-26s) présentant un ensemble de microélectrodes (21) ;
- nettoyage (E2) du support par plasma ;
- culture (E3) d'îlots (230) de Langerhans sur l'ensemble de microélectrodes (21).

15. Utilisation d'un capteur selon la revendication 1 pour :
- une étude physiologique pour un criblage de molécules toxiques ; ou
- une étude physiologique à visée thérapeutique ; ou
- une étude physiologique pour suivre la différentiation de cellules à partir de cellules souches en cellules β-pancréatiques.

## Patentansprüche

1. Sensor (2), der Folgendes umfasst:
- eine Einheit von Mikroelektroden (21);
**dadurch gekennzeichnet, dass** er auch Folgendes umfasst:
- Langerhans-Inseln (230) in Kultur auf der Einheit von Mikroelektroden (21);
wobei die Einheit von Mikroelektroden (21) angepasst ist, um dynamisch in Echtzeit und kontinuierlich elektrische Signale (V), die von den Langerhans-Inseln (230) bei ihrer physiologischen Aktivierung erzeugt werden, zu messen,
und dadurch, dass er außerdem mindestens eine Einheit von Verarbeitungseinheiten (25) umfasst, wobei jede Verarbeitungseinheit (25) mit einer Mikroelektrode (21) gekoppelt und angepasst ist
- für das Formen der gemessenen elektrischen Signale (V); und
- das Extrahieren mindestens eines Parameters (P), der einen Insulinbedarf eines Patienten (9) in Echtzeit und kontinuierlich wiedergibt.

2. Sensor (2) nach Anspruch 1, wobei jede Verarbeitungseinheit (25) eine Subeinheit (251) umfasst, die angepasst ist, um das Formen der elektrischen Signale (V) auszuführen, wobei die Subeinheit (251) angepasst ist, um die elektrischen Signale (V) zu empfangen und sie gemäß mathematischen Funktionen vom Typ Verstärkung und/oder Filtern zu verarbeiten.

3. Sensor (2) nach einem der Ansprüche 1 bis 2, wobei die Verarbeitungseinheiten (25) Schaltungen sind, die nach Maß in analoger Mikroelektroniktechnologie ausgelegt sind.

4. Sensor (2) nach einem der vorhergehenden Ansprüche, wobei jede Verarbeitungseinheit (25) eine Subeinheit (253) umfasst, die angepasst ist, um das Extrahieren des Parameters (P) auszuführen, wobei der Wert des Parameters ausgehend von Eingangssignalen der Subeinheit (253), die die geformten elektrischen Signale (V) umfasst, und einem Sollwert (Vc) berechnet wird.

5. Sensor (2) nach einem der Ansprüche 1 bis 4, wobei die Einheit von Mikroelektroden (21) angepasst ist, um elektrische Signale in Form elektrischer Potenziale zu messen.

6. Sensor (2) nach einem der Ansprüche 1 bis 5, wobei die Verarbeitungseinheit (25) angepasst ist, um ein Aktionspotenzial zu erfassen.

7. Sensor (2) nach einem der Ansprüche 1 bis 6, der außerdem einen Regler (26) umfasst, um den Parameter (P), der den Insulinbedarf wiedergibt, in ein Steuersignal (C) zum Steuern eines Insulinverteilers (3) umzuwandeln.

8. Sensor (2) nach einem der Ansprüche 1 bis 7, der außerdem einen Halbleiterträger (21s, 21-25s, 21-25-26s) umfasst, der mindestens die Einheit von Mikroelektroden (21) umfasst.

9. Sensor (2) nach einem der Ansprüche 1 bis 8, der außerdem eine halbdurchlässige Membran (27) umfasst, die angepasst ist, um
- eine Flüssigkeit zu filtern, die zu den Langerhans-Inseln (230) Zugang haben soll, und
- die Moleküle der Flüssigkeit, deren Gewicht größer ist als 65 kDa, zu blockieren.

10. Sensor (2) nach einem der Ansprüche 1 bis 9, wobei die Langerhans-Inseln (230) porciner, muriner oder menschlicher Herkunft sind.

11. Vorrichtung (1), die einen Insulinverteiler (3) zum Verteilen einer Insulinmenge in dem Körper eines Patienten (9) umfasst,
wobei die Vorrichtung (1) **dadurch gekennzeichnet ist, dass** sie außerdem einen Sensor (2) nach einem der Ansprüche 1 bis 10 umfasst.

12. Vorrichtung (1) nach Anspruch 11, wobei der Sensor (2) einen Regler (26) umfasst, um den Parameter (P), der den Insulinbedarf wiedergibt, in ein Steuersignal (C) zum Steuern des Insulinverteilers (3) umzuwandeln.

13. Vorrichtung (1) nach Anspruch 12, wobei die Einheit von Mikroelektroden (21), der Regler (26) und die Einheit von Einheiten (25) auf ein und demselben Halbleiterträger hergestellt oder auf ihm angefügt sind.

14. Herstellungsverfahren eines Sensors (2) nach einem der Ansprüche 1 bis 10, **dadurch gekennzeichnet, dass** es die folgenden Schritte erfasst:
- Liefern (E1) eines Trägers (21s, 23-25s, 23-25-26s), der eine Einheit von Mikroelektroden (21) aufweist;
- Reinigen (E2) des Trägers durch Plasma;
- Kultur (E3) von Langerhans-Inseln (230) auf der Einheit von Mikroelektroden (21).

15. Gebrauch eines Sensors nach Anspruch 1, für:
- eine physiologische Studie für ein Screenen toxischer Moleküle; oder
- eine physiologische Studie mit therapeutischer Zweckbestimmung; oder
- eine physiologische Studie zur Überwachung der Unterscheidung von Zellen ausgehend von Zellstämmen in Pankreas-β-Zellen.

## Claims

1. Sensor (2) including:
- a set of microelectrodes (21);
**characterized in that** it also comprises
- islets (230) of Langerhans in culture on the set of microelectrodes (21);
the set of microelectrodes (21) being suitable for dynamically measuring, in real time and continuously, electrical signals (V) produced by the islets (230) of Langerhans during their physiological activation,
and **in that** it further comprises at least one set of processing units (25), in which each processing unit (25) is coupled to a microelectrode (21) and suitable for:
- forming the electrical signals (V) measured; and
- extracting at least one parameter (P) indicating a need for insulin by a patient (9), in real time and continuously.

2. Sensor (2) according to claim 1, in which each processing unit (25) comprises a sub-unit (251) suitable for forming the electrical signals (V), with the sub-unit (251) being suitable for receiving the electrical signals (V) and processing them according to amplification and/or filtering-type mathematical functions.

3. Sensor (2) according to any of claims 1 to 2 in which the processing units (25) are customized analog microelectronic circuits.

4. Sensor (2) according to any of the preceding claims, in which each processing unit (25) comprises a sub-unit (253) suitable for extracting the parameter (P), with the value of the parameter being calculated on the basis of input signals of the sub-unit (253) comprising the electrical signals (V) formed and a setpoint value (Vc).

5. Sensor (2) according to any of claims 1 to 4 in which the set of microelectrodes (21) is suitable for measuring electrical signals in the form of electric potentials.

6. Sensor (2) according to any of claims 1 to 5, in which the processing unit (25) is suitable for detecting an action potential.

7. Sensor (2) according to any of claims 1 to 6, also including a regulator (26) for transforming the parameter (P) indicating the need for insulin into a control signal (C) for controlling an insulin dispenser (3) .

8. Sensor (2) according to any of claims 1 to 7, also including a semiconductor substrate (21s, 21-25s, 21-25-26s) comprising at least the set of microelectrodes (21).

9. Sensor (2) according to any of claims 1 to 8, also including a semi-permeable membrane (27), suitable for
- filtering a liquid needing access to the islets (230) of Langerhans, and
- blocking the molecules of said liquid of which the weight is greater than 65kDa.

10. Sensor (2) according to one of claims 1 to 9, in which the islets (230) of Langerhans are of porcine, murine or human origin.

11. Device (1) including an insulin dispenser (3) for dispensing an amount of insulin into a patient's body (9),
which device (1) is **characterized in that** it also includes a sensor (2) according to one of claims 1 to 10.

12. Device (1) according to claim 11 in which the sensor (2) includes a regulator (26) for transforming the parameter (P) indicating the need for insulin into a control signal (C) for controlling the insulin dispenser (3).

13. Device (1) according to claim 12 in which the set of microelectrodes (21), the regulator (26) and the set of units (25) are produced or placed on the same semiconductor substrate.

14. Method for producing a sensor (2) according to any of claims 1 to 10, **characterized in that** it includes the following steps:
- providing (E1) a substrate (21s, 23-25s, 23-25-26s) having a set of microelectrodes (21);
- cleaning (E2) the substrate with plasma;
- culturing (E3) islets (230) of Langerhans on the set of microelectrodes (21).

15. Use of a sensor according to claim 1 for:
- a physiological study for screening toxic molecules; or
- a physiological study for a therapeutic end; or
- a physiological study for monitoring the differentiation of cells from stem cells into β-pancreatic cells.
